(19) **Europäisches Patentamt / European Patent Office / Office européen des brevets**

(11) **EP 4 679 092 A1**

(12) # EUROPEAN PATENT APPLICATION

(43) Date of publication:
**14.01.2026 Bulletin 2026/03**

(21) Application number: **24187247.2**

(22) Date of filing: **08.07.2024**

(51) International Patent Classification (IPC):
**G01N 33/564** (2006.01)     **G01N 33/569** (2006.01)
**G01N 33/68** (2006.01)

(52) Cooperative Patent Classification (CPC):
**G01N 33/56994; G01N 33/564; G01N 33/6854**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Charité - Universitätsmedizin Berlin**
  **10117 Berlin (DE)**
• **The Johns Hopkins University**
  **Baltimore, MD 21218 (US)**

(72) Inventors:
• **KREYE, Jakob**
  **14469 Potsdam (DE)**
• **LARMAN, Harry**
  **Baltimore, 5423 (US)**

(74) Representative: **Schulz Junghans
Patentanwälte PartGmbB
Großbeerenstraße 71
10963 Berlin (DE)**

Remarks:
The complete document including Reference Table(s) and the Sequence Listing(s) can be downloaded from the EPO website

(54) **BIOMARKERS FOR HERPES-SIMPLEX VIRUS AUTOIMMUNITY**

(57)     The invention relates to a method to measure the level of HSV1-specific antibodies in a biological sample, and methods to stratify herpes simplex encephalitis patients according to their risk of developing autoimmune anti-NMDA-receptor encephalitis. The invention further encompasses pharmaceutical formulations, and methods of treatment useful for treating encephalitis patients.

**Description**

Background

Anti-NMDA-receptor encephalitis (NMDARE) is the most common form of autoimmune encephalitis, typically presenting with psychosis, seizures, cognitive impairment, and autonomic dysfunction (Graus et al., 2016). The autoimmune disease is mediated via autoantibodies directed against the NMDAR leading to receptor internalization, subsequent NMDAR dysfunction, and profound synaptic changes (Hughes et al., 2010; Kreye et al., 2016; Planaguma et al., 2015). These alterations commonly cause a severe clinical phenotype often requiring intensive care treatment and mechanical ventilation, with mortality rate estimated up to 7% (Nosadini et al., 2021; Titulaer et al., 2013). However, immunosuppressive therapies lead to marked improvement in majority of patients, with rapid initiation being associated to better outcome (Titulaer et al., 2013).

In a subgroup of NMDARE patients, disease onset occurs within a few months after herpes simplex encephalitis (HSE) (Armangue et al., 2018; Pruss et al., 2012). According to current practice, diagnosis is based on presentation of clinical symptoms such as seizures, movement disorders, or other focal neurological deficits and on autoantibody detection from cerebrospinal and blood. A lumbar puncture may be carried out and assessed for inflammatory indicators such as lymphocyte number, antibody levels, or inflammatory proteins, together with tests to rule out an infectious pathogen.

Accurate and early detection of HSE patient with a high risk of developing NMDARE could improve outcome via increased awareness, facilitating closer clinical follow-ups with pathological antibody diagnostics, providing a pathway to earlier diagnosis and immunosuppressive treatments (Titulaer et al., 2013). A prospective study had shown that about 23% of HSE patients develop NMDARE, however at present it is not possible to identify the patients in whom autoimmune sequalae will arise. HSE patients who develop NMDARE display worse outcomes at 6 and 12 months after onset compared to HSE patients without NMDARE, as measured in a modified ranking scale (Armangue et al., 2023).

The underlying mechanisms and risk factors for the development of secondary autoimmunity after primary infectious disease have so far remained widely unknown. A previous study indicated an increased interferon response in HSE followed by NMDARE patients when compared to an HSE without NMDARE group (Armangue et al., 2023), suggesting a hyperactivated immune response in the respective patients. However, there was no clear threshold to distinguish the group by their interferon response and the fact that the difference was only found at in one out of four investigated time points suggests a narrow diagnostic window.

Based on the above-mentioned state of the art, the objective of the present invention is to provide robust means and methods to predict the prognosis of HSE patients, and accurately identify HSE patients likely to develop HSV-driven NMDARE autoimmunity. This objective is attained by the subject-matter of the independent claims of the present specification, with further advantageous embodiments described in the dependent claims, examples, figures and general description of this specification.

Summary of the Invention

The inventors use phage immunoprecipitation sequencing (PhIP-Seq) to identify distinct antibody reactivities to Herpes simplex virus Type 1 (HSV1) antigens with the potential to predict NMDARE cases arising after HSE more accurately than existing methods. New methods based on this finding provide a basis for enhanced monitoring for development of neurological symptoms in at-risk patients, and may form the basis of targeted therapeutic interventions described herein.

A first aspect of the invention relates to methods of measuring HSV1-specific antibodies in a biological sample. The method comprises of measuring the level of antibodies specific for at least one of the following predictive antigens derived from HSV1, the UL42 region polypeptide SEQ ID NO 001, the UL48 region polypeptide SEQ ID NO 002, the UL42 peptides SEQ ID NO 003, 004, and 005, or the UL48 peptides SEQ ID NO 006 and 007. In some embodiments, the method involves measuring the global antibody reactivity level in a biological sample to either of the longer polypeptide regions SEQ ID NO 002 or SEQ ID NO 003 comprising 3 and 2 overlapping peptide B epitopes from the UL42 and UL48 proteins, respectively. In other embodiments, the method involves measuring the level of antibodies specific for at least one of the individual peptides SEQ ID NO 003-007, each of which has been shown by the inventors to be a statistically significant biomarker predicting development of NMDARE following an episode of HSE in individual patients.

The method according to this aspect of the invention involves measuring the level of antibodies specific for an HSVI antigen present in a biological sample. This assay has clinical relevance for stratifying HSE patient according to their likely prognosis. The invention further provides polypeptides and peptides of use as capture antigens in methods to detect the level of antibodies specific to HSV described in the preceding paragraph. A capture antigen of use to measure the level of antibodies specific for SEQ ID NO 001-007 is similar in sequence and/or conformation in order to retain the same antibody-binding epitopes.

Capture antigens of use as reagents in assays to detect the level of antibodies specific for SEQ ID NO 001, and SEQ ID NO 003-5 include sequences derived from the HSV1 DNA polymerase processivity factor, also referred to as the UL42 protein, as well as variants thereof likely to have the same confirmation and antibody-binding qualities.

A first set of useful HSV antigens include a UL42 region polypeptide comprising or consisting of SEQ ID NO 001, or alternatively a UL42 B cell peptide epitope (a B cell epitope peptide between 5 and 80 amino acids in length comprised within the longer polypeptide SEQ ID NO 001, such as SEQ ID NO 003, 004, or 005). In some embodiments, the method to detect HSV-specific antibodies uses a variant UL42 region polypeptide, or a variant UL42 peptide with at least 95% sequence identity compared to SEQ ID NO 001, 003, 004, or 005.

A second group of useful HSV antigens are derived from the HSV1 Tegument protein, also called the UL48 protein. These potential capture antigens include a UL48 region polypeptide comprising, or consisting of SEQ ID NO 002 (337 to 420 of UL48 Uniprot P06492), or in some embodiments a UL48 B cell peptide epitope (a B cell epitope peptide between 5 and 80 amino acids in length comprised within the longer polypeptide SEQ ID NO 002, such as SEQ ID NO 005, or 006). In some embodiments, the method uses a variant UL42 region polypeptide, or a variant UL48 peptide with at least 95% sequence identity SEQ ID NO 002, SEQ ID NO 005, or 006 as a capture antigen in a method to measure antibodies specific to SEQ ID NO 002, 005, or 006.

Measuring antigen specific antibodies in liquid patient samples such as plasma, serum, or cerebral spinal fluid (CSF) can be carried out by various analytic biochemistry methods known to the skilled person, such as enzyme linked immunosorbent assay using plates coated with said antigens, or multiplex assay techniques where antigen is conjugated to cytometric beads assays prior to analysis by flow cytometry. In particular embodiments, the method assays antibody reactivity to antigens from both the first and second groups of biomarkers, as antibodies specific for either the UL42 region polypeptide, and/or the UL48 region polypeptide can provide a basis for predicting whether a patient develops NMDARE following HSE.

Another aspect of the invention are methods, or systems to predict the prognosis of a patient diagnosed with HSE, comprising performing the method as specified in the preceding 4 paragraphs and then assigning the patient a high likelihood of developing NMDARE to an HSE patient if the assay detects a level of any antibodies specific for at least one HSV antigens above a threshold. A low likelihood of developing HSV-induced NMDARE is assigned if no antibodies specific for any of the HSV antigens described above are detected in the patient sample.

HSE patients who develop NMDARE display worse outcomes at 6 and 12 months after onset compared to HSE patients without NMDARE (Armangue et al., 2023). In some embodiments, the patient having a level of antibodies for at least one HSV antigen according to the invention above a threshold, is assigned a high likelihood of a poor clinical outcome. In some embodiments, the patient having a level of antibodies for any HSV antigen according to the invention below a threshold, is assigned a low likelihood of a worse clinical outcome in the 6 to 13 months following HSE onset.

Another aspect of the invention relates to immunomodulatory agents, namely immunosuppressive drugs, or intravenous human immunoglobulin (IVIG) preparations for use in a subset of patients having been diagnosed previously with HSE, and having been determined to have a high risk of developing NMDARE according to the method of the preceding paragraph.

Another aspect of the invention is a method of treating a patient assigned a high likelihood of developing NMDAR encephalitis according to the methods herein, comprising administering the patient an effective dose of an immunosuppressive agent, or an intravenous immunoglobulin infusion (IVIG), and/or performing plasmapheresis.

Another aspect of the invention is a kit, or a device for HSE samples comprising any one of the HSV antigens as specified above, and optionally, a molecular probe specific for human Ig conjugated to a detectable label for measuring the level of antibodies reactive to the HSV antigens in a patient sample.

*Terms and definitions*

*General*

For purposes of interpreting this specification, the following definitions will apply and whenever appropriate, terms used in the singular will also include the plural and vice versa. If any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one, or both limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term PBS in the context of the present specification relates to Phosphate Buffered Saline: NaCl: 8.0 g/L; KCl: 0.2 g/L; $Na_2HPO_4$: 1.44 g/L $KH_2PO_4$: 0.24 g/L, pH 7.4.

*Herpes simplex encephalitis and NMDAR encephalitis*

The term herpes simplex encephalitis (HSE), in the context of the present specification relates to a type of meningitis that arises due to a HSV infection. At present, diagnosis is based on clinical history, physical exam (symptoms include fever, headache, stiffness, altered mental status, or behavioral changes). If HSE is suspected, a sample of cerebrospinal fluid (CSF) may be collected via lumbar puncture and examined for signs of infection such as elevated lymphocyte counts, and/or measurement of the expression level of viral genes by PCR.

The term *N-methyl-D-aspartate receptor* (also known as the *NMDA receptor* or *NMDAR*) refers to the glutamate receptors acting as ion channels expressed by human cells such as neurons. The term includes several heterotetrametric formats comprising different combinations of the receptor subunits GluN1, GluN2, and GluN3, encoded by the *GRIN* gene family.

*Anti-NMDA receptor encephalitis,* or *NMDARE* is an umbrella term referring to encephalitis characterized by antibody-driven pathology, which can be subdivided into patients with a preceding episode of HSE (also sometimes referred to in this text as NMDARE-pHSE) and those without a clinical history of HSE (NMDARE-other).

The term *herpes simplex virus* in the context of the present specification relates to the viral pathogen of the Family *Ortho Herpesviridae,* Genus *Simplex virus* associated with both latent (non-symptomatic), or lytic infections (with a range of mild to severe symptoms). There are two types of HSV, Type 1 and Type 2 (HSV1, HSV2). *Human alpha herpesvirus* is an alternative name for HSV.

The term *UL42* in the context of the present specification relates to the protein encoded by the HSV1 gene *UL42,* also known as DNA polymerase processivity factor (Uniprot P10226).

The term *UL48* in the context of the present specification relates to the protein encoded by the human gene *UL48,* also known as tegument protein VP14 (Uniprot P06492).

*Sequences*

Sequences similar or homologous (e.g., at least about 70% sequence identity) to the sequences disclosed herein are also part of the invention. In some embodiments, the sequence identity at the amino acid level can be about 80%, 85%, 90%, 91%, 92%, 93%, 94%, 95%, 96%, 97%, 98%, 99% or higher. At the nucleic acid level, the sequence identity can be about 70%, 75%, 80%, 85%, 90%, 91%, 92%, 93%, 94%, 95%, 96%, 97%, 98%, 99% or higher. Alternatively, substantial identity exists when the nucleic acid segments will hybridize under selective hybridization conditions (e.g., very high stringency hybridization conditions), to the complement of the strand. The nucleic acids may be present in whole cells, in a cell lysate, or in a partially purified or substantially pure form.

In the context of the present specification, the terms *sequence identity* and *percentage of sequence identity* refer to a single quantitative parameter representing the result of a sequence comparison determined by comparing two aligned sequences position by position. Methods for alignment of sequences for comparison are well-known in the art. Alignment of sequences for comparison may be conducted by the local homology algorithm of Smith and Waterman (Smith & Waterman, 1981), by the global alignment algorithm of Needleman and Wunsch (Needleman & Wunsch, 1970), by the search for similarity method of Pearson and Lipman (Pearson & Lipman, 1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http://blast.ncbi.nlm.nih.gov/).

One example for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment. One such example for comparison of nucleic acid sequences is the BLASTN algorithm that uses the default settings: Expect threshold: 10; Word size: 28; Max matches in a query range: 0; Match/Mismatch Scores: 1.-2; Gap costs: Linear. Unless stated otherwise, sequence identity values provided herein refer to the value obtained using the BLAST suite of programs (Altschul et al., 1990) using the above identified default parameters for protein and nucleic acid comparison, respectively.

Reference to identical sequences without specification of a percentage value implies 100% identical sequences (i.e. the same sequence). Particular embodiments make use of the sequences as disclosed herein (i.e. 100% identical).

*General Biochemistry: Peptides, Amino Acid Sequences*

The term *polypeptide* in the context of the present specification relates to a molecule consisting of 81 or more amino acids that form a linear chain wherein the amino acids are connected by peptide bonds. The amino acid sequence of a polypeptide may represent the amino acid sequence of a whole (as found physiologically) protein, or fragments thereof. The term "polypeptides" and "protein" are used interchangeably herein and include proteins and fragments thereof. Polypeptides are disclosed herein as amino acid residue sequences.

The term *peptide* or in the context of the present specification relates to a molecule consisting of up to 80 amino acids, in particular 5 to 60 amino acids, more particularly 20 to 60 amino acids, that form a linear chain wherein the amino acids are connected by peptide bonds.

Amino acid residue sequences are given from amino to carboxyl terminus. Capital letters for sequence positions refer to L-amino acids in the one-letter code (Stryer, Biochemistry, 3rd ed. p. 21). Lower case letters for amino acid sequence positions refer to the corresponding D- or (2R)-amino acids. Sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

The term *variant* refers to a polypeptide that differs from a reference polypeptide, but retains the same essential biochemical properties. A typical variant of a polypeptide differs in its primary amino acid sequence from another, reference polypeptide. Generally, differences are limited so that the sequences of the reference polypeptide and the variant are closely similar overall and, in many regions, identical. A variant and reference polypeptide may differ in amino acid sequence by one or more modifications (e.g., substitutions, additions, and/or deletions). A substituted or inserted amino acid residue may, or may not be, one encoded by the genetic code. A variant of a polypeptide may be naturally occurring such as an allelic variant, or it may be a variant that is not known to occur naturally. Preferably the variant comprises no more than 1 amino acid substitution for every 10 amino acids in length of the reference sequence, though these need not be arranged regularly and may be placed anywhere in the reference sequence.

The term *conservative amino acid substitution* in the context of the present specification refers to replacing an amino acid in a peptide or polypeptide with a different amino acid with similar biochemical properties. This is sometimes referred to as an amino acid replacement, or a conservative mutation. In particular embodiments, a conservative amino acid substitution in one of the HSV antigen sequences provided herein is according to the following substitution rules:

- glycine (G) and alanine (A) are interchangeable; valine (V), leucine (L), and isoleucine (I) are interchangeable, A and V are interchangeable;

- tryptophan (W) and phenylalanine (F) are interchangeable, tyrosine (Y) and F are interchangeable;

- serine (S) and threonine (T) are interchangeable;

- aspartic acid (D) and glutamic acid (E) are interchangeable

- asparagine (N) and glutamine (Q) are interchangeable; N and S are interchangeable; N and D are interchangeable; E and Q are interchangeable;

- methionine (M) and Q are interchangeable;

- cysteine (C), A and S are interchangeable;

- proline (P), G and A are interchangeable;

- arginine (R) and lysine (K) are interchangeable

In the context of the present specification, the term *amino acid linker* or *peptide linker* refers to a polypeptide of variable length that is used to connect two polypeptides, to generate a single chain polypeptide. Exemplary embodiments of linkers useful for practicing the invention specified herein are oligopeptide chains consisting of 1, 2, 3, 4, 5, 10, 20, 30, 40 or 50 amino acids, which may link 2 or more HSV peptide or polypeptide antigens described herein.

There is no constraint on the amino acid composition of a peptide linker. In certain embodiments, the linker consists of amino acids selected from the group of G S, A and D. An important characteristic of the conjugate peptide linkers as specified above is low immunogenicity. In particular desirable embodiments of the domain peptide linkers specified above, the sequences are primarily made up of stretches of amino acids such as glycine (G) and serine (S). In certain embodiments peptide linker is $\geq 15$ amino acids in length, particularly 15 to 30 amino acids in length wherein the amino acids are selected from G S, A and D. A non-limiting example of an amino acid linker is a monomer or di-, tri- or tetramer of a peptide motif composed of three or four glycine and one serine.

*Cell Biology, diagnostic method inventions: Markers, ligands*

In the present specification, the level of HSV antibody may be assayed via techniques such as fluorescence microscopy, flow cytometry, ELISPOT, ELISA or multiplex analyses.

The term *molecular probe* in the context of the present specification relates to a specific ligand, particularly an antibody, antibody fragment, an antibody-like molecule or aptamer, more particularly an antibody or antibody fragment, that can bind to the target molecule, human Ig, with a dissociation constant of $\leq 10^{-7}$ mol/l, particularly $\leq 10^{-9}$ mol/l. The molecular probe comprises a detectable label such as a particle, bead, dye, or enzyme.

The term *Detectable Label* or *detectable marker the* context of the present specification relates to a molecule, with distinctive properties that is conjugated or attached to a carrier molecule, such as a ligand (molecular probe), more specifically the ligand being an antibody, to facilitate the detection and quantification of the ligand's target in a biological sample. The label, or marker allows visual or instrumental identification of the presence (qualitative measurement), or concentration (quantitative measurement) of the target molecule. Common types of detectable labels used in procedures such as ELISA and other diagnostic methods include enzymes, fluorescent molecules, fluorescent dye, radioactive isotopes, colloidal gold particles, and biotin, among others. These labels emit signals or exhibit distinct properties that can be measured, or visualized, to determine the presence and/or quantity of the target molecule in the sample.

The term *fluorescent dye* in the context of the present specification relates to a small molecule capable of fluorescence in the visible, or near infrared spectrum. Examples of fluorescent labels or labels presenting a visible color include, without being restricted to, fluorescein isothiocyanate (FITC), rhodamine, allophycocyanin (APC), peridinin chlorophyll (PerCP), phycoerythrin (PE), Alexa Fluors (Life Technologies, Carlsbad, CA, USA), dylight fluors (Thermo Fisher Scientific, Waltham, MA, USA) ATTO Dyes (ATTO-TEC GmbH, Siegen, Germany), BODIPY Dyes (4,4-difluoro-4-bora-3a,4a-diaza-s-indacene based dyes).

*Binding; Binders, Ligands, Antibodies:*

If not specified more narrowly in the *Detailed Description of the Invention,* reference to binders and ligands encompasses antibodies, antibody-like molecules and aptamers as defined in the following paragraphs.

The term *specific binding* in the context of the present invention refers to a property of ligands that bind to their target with a certain affinity and target specificity. The affinity of such a ligand is indicated by the dissociation constant of the ligand. A specifically reactive ligand has a dissociation constant of $\leq 10^{-8}$ mol/L (particularly $\leq 10^{-9}$ mol/L) when binding to its target, but a dissociation constant at least three orders of magnitude higher in its interaction with a molecule having a globally similar chemical composition as the target, but a different three-dimensional structure.

In the context of the present specification, the term *dissociation constant ($K_D$)* is used in its meaning known in the art of chemistry and physics; it refers to an equilibrium constant that measures the propensity of a complex composed of [in most cases, two] different components to dissociate reversibly into its constituent components. The complex can be e.g. an antibody-antigen complex AbAg composed of antibody Ab and antigen Ag. $K_D$ is expressed in molar concentration [mol/l] and corresponds to the concentration of [Ab] at which half of the binding sites of [Ag] are occupied, in other words, the concentration of unbound [Ab] equals the concentration of the [AbAg] complex. The dissociation constant can be calculated according to the following formula:

$$K_D = \frac{[Ab] * [Ag]}{[AbAg]}$$

[Ab]: concentration of antibody; [Ag]: concentration of antigen; [AbAg]: concentration of antibody-antigen complex

In the context of the present specification, the terms *off-rate* ($K_{off}$;[1/sec]) and *on-rate* ($K_{on}$; [L/(sec*mol)]) are used in their meaning known in the art of chemistry and physics; they refer to a rate constant that measures the dissociation ($K_{off}$) or association ($K_{on}$) of an antibody with its target antigen. $K_{off}$ and $K_{on}$ can be experimentally determined using methods well established in the art. A method for determining the $K_{off}$ and $K_{an}$ of an antibody employs surface plasmon resonance. This is the principle behind biosensor systems such as the Biacore® or the ProteOn® system. They can also be used to determine the dissociation constant $K_D$ by using the following formula:

$$K_D = \frac{[K_{off}]}{[K_{on}]}$$

The natural upper limit for the on-rate $K_{on}$ is $10^9$ L/(sec*mol).

In the context of the present specification, the term *antibody* refers to whole antibodies including but not limited to immunoglobulin type G (IgG), type A (IgA), type D (IgD), type E (IgE) or type M (IgM), any antigen-binding fragment or single chains thereof and related or derived constructs. A whole antibody is a glycoprotein comprising at least two heavy (H) chains and two light (L) chains inter-connected by disulfide bonds. Each heavy chain is comprised of a heavy chain variable region ($V_H$) and a heavy chain constant region ($C_H$). The heavy chain constant region of IgG is comprised of three domains, $C_H1$, $C_H2$ and $C_H3$. Each light chain is comprised of a light chain variable region (abbreviated herein as $V_L$) and a light chain constant region ($C_L$). The light chain constant region is comprised of one domain, $C_L$. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component of the classical complement system. Similarly, the term encompasses a so-called nanobody or single domain antibody, an antibody fragment consisting of a single monomeric variable antibody domain.

The term *aptamer* relates to an oligonucleotide or peptide molecule that binds to a specific target molecule. Aptamers can be created by selecting them from a large random sequence pool. Nucleic acid aptamers can be generated through repeated rounds of in-vitro selection or equivalently, SELEX (systematic evolution of ligands by exponential enrichment) to bind to molecular targets such as small molecules, proteins, or nucleic acids through non-covalent interactions. Aptamers offer molecular recognition properties that rival that of antibodies.

*Immunomodulatory therapy*

In the context of the present specification, the term *immunomodulatory therapy* is meant to encompass formulations reducing symptoms associated with antibody-driven pathology including, but not limited to, intravenous immunoglobulin (IVIG) CAS 9007-8304, as well as immunosuppressive agents (drugs) acting on immune cells in general, or specifically acting to reduce B cell and/or antibody production.

In the context of the present specification, the term *immunosuppressive agent* encompasses drugs which reduce the activity of the immune system in general, or specifically reduce antibody-mediated pathology. Examples include, but are not limited to, steroidal antiinflammatory or corticosteroids (e.g. prednisone CAS 53-03-2, methyl prednisone CAS 83-43-2), or immunosuppressant medications targeting inflammatory pathways such as azathioprine CAS 44-86-6, and mycophenolate mofetil CAS 128794-94-5. Further embodiments include cell-growth inhibitors and chemotherapy agents such as bortezomib CAS 129324-69-7, cyclophosphamide CAS 50-18-0, and methotrexate CAS 59-05-2. The term further encompasses monoclonal antibody drugs depleting B cells such as rituximab CAS 174722-31-7, inebilizumab CAS 1299440-37-1, or alemtuzumab CAS 216503-57-0 and/or targeting pro-inflammatory molecules like the interleukin-6 receptor antibody tocilizumab CAS 357823-41-9.

As used herein, the term *treating* or *treatment* of any disease or disorder (e.g. NMDARE) refers in one embodiment to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral, or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624). The invention also encompasses nanoparticles, liposomes, or cellular carriers within the meaning of *pharmaceutically acceptable carrier.*

Detailed Description of the Invention

In the months and years following recovery from HSE, a subset 23% of patients develop a secondary autoimmune encephalitis, NMDARE (Armangue et al., 2023) associated with a very high likelihood of adverse outcomes. The inventors identified HSV1 polypeptides and peptide antigens targeted by antibodies found almost exclusively in blood, or cerebrospinal fluid biological samples of HSE patients who later developed NMDARE. Methods to detect such antibodies in biological samples obtained from human subjects are of use to identify high-risk patients before symptoms develop, facilitating enhanced monitoring procedures, and providing a potential for clinical interventions at the earliest time point of disease onset.

*Methods to detect HSV1-specific antibodies*

A first aspect of the invention relates to a method to detect HSV1-specific antibodies. The method may be applied to a biological sample comprising antibodies which has been obtained from a patient whose symptoms and viral load indicate a diagnosis of herpes simplex virus encephalitis (HSE).

Methods according to the first aspect of the invention comprise measuring the level of antibodies in a biological sample that are specific for at least one HSV antigen selected from, or contained within at least one of two antigenic regions identified by the inventors in two different HSV1 proteins. The inventors observed that antibodies in samples from HSE patients who later developed NMDARE bound to peptides within at least one, and sometimes both of the following antigenic regions.

In certain embodiments, the method according to the first aspect invention measures the level of antibodies specific for a polypeptide region derived from the UL42 protein of HSV1 (Uniprot P10226), the processivity subunit of HSV1 DNA polymerase. In some embodiments, the method detects antibodies specific for a UL42 region polypeptide comprising SEQ ID NO 001 (amino acid residues of 393 to 488 of UL42). This region of UL42 contains three overlapping peptides targeted by antibodies in NMDAR high-risk patients, SEQ ID NO 003, 004, and 005. In particular embodiments, the method detects the presence of antibodies specific for a polypeptide consisting of SEQ ID NO 001.

In some embodiments, a variant UL42 polypeptide at least 95% identical to SEQ ID NO 001 is provided for use as a capture antigen in a method to detect antibodies specific to the UL42 polypeptide region SEQ ID NO 001. In certain embodiments, the method involves measuring the level of antibodies in a sample binding to a variant UL42 region polypeptide comprising or consisting of a polypeptide having at most 1 amino acid substitution, insertion, or deletion for every 10 amino acids in length compared to SEQ ID NO 001. Such amino acid substitutions are preferably conservative amino acid substitutions that provide enhanced biochemical properties of the polypeptide such as increased stability, or adhesion to assay surfaces, while maintaining a confirmation similar to the wildtype polypeptide of SEQ ID NO 001 to preserve the antibody-binding profile.

Linear B cell epitopes vary from 5 to 80 amino acids in length, such that the region SEQ ID NO 001 comprises a large number of clinically informative overlapping B cell epitopes of use as HSV antigens in methods according to the invention. In certain embodiments of the method, the level of antibodies specific for SEQ ID NO 001, comprises measuring the level of antibodies binding a UL42 peptide comprising 5 to 80 consecutive amino acids comprised within the longer polypeptide region SEQ ID NO 001 in the sample. In particular embodiments the method measures antibodies binding to at least 3 overlapping UL42 peptides derived from SEQ ID NO 001, each having a length of about 20 to 60 amino acids.

In some embodiments, a variant UL48 polypeptide at least 95% identical to SEQ ID NO 002 is provided for use as a capture antigen in a method to detect antibodies specific to the UL48 region polypeptide SEQ ID NO 002. In certain embodiments, the method involves measuring the level of antibodies in a sample binding to a variant UL42 peptide comprising, or consisting of a peptide having at most 1 amino acid substitution, insertion, or deletion for every 10 amino acids in length compared to any UL42 peptide comprising, or consisting of 5 to 80 consecutive amino acids comprised within the longer polypeptide SEQ ID NO 001, such that the variant UL42 peptide maintains the antibody epitope conformation of the template sequence.

In certain embodiments, the method according to the first aspect invention measures the level of antibodies specific for a UL48 region polypeptide comprising SEQ ID NO 002 (337 to 420 of UL48 Uniprot P06492). This region of UL48 comprises peptides targeted by antibodies in different NMDAR high-risk patients, SEQ ID NO 006, and 007. In particular embodiments, the method detects the level of antibodies specific for a polypeptide consisting of SEQ ID NO 002.

In certain embodiments, the method involves measuring the level of antibodies in a HSE patient sample binding to a variant UL48 region polypeptide comprising or consisting of a polypeptide having at most 1 amino acid substitution, insertion, or deletion for every 10 amino acids in length compared to SEQ ID NO 002. In particular embodiments, the variant UL48 polypeptide comprises only conservative amino acid substitutions compared to SEQ ID NO 002.

In certain embodiments of the method according to the first aspect of the invention, the level of antibodies specific for SEQ ID NO 002 comprises measuring the level of antibodies binding at least one UL48 peptide comprising 5 to 80 consecutive amino acids comprised within the longer polypeptide region SEQ ID NO 002 is measured in a sample obtained from a patient. In particular embodiments the method measures antibodies binding to at least 3 UL48 peptides derived from SEQ ID NO 002, each having a length of about 20 to 60 amino acids. In certain embodiments, the method involves measuring the level of antibodies in a sample binding to a variant UL42 peptide used as a capture antigen, comprising, or consisting of a peptide having at most 1 amino acid substitution, insertion, or deletion for every 10 amino acids in length compared to any UL42 peptide comprising, or consisting of 5 to 80 consecutive amino acids comprised within the longer polypeptide SEQ ID NO 001.

In certain embodiments of the method according to the invention, the method comprises measuring the level of antibodies specific for a capture antigen selected from a UL48 region polypeptide comprising SEQ ID NO 002 or a variant UL48 region polypeptide comprising a polypeptide having up to 5 amino acids substitutions, insertions, or deletions compared to SEQ ID NO 002. In certain embodiments, the UL48 region polypeptide consists of SEQ ID NO 002.

In certain embodiments of the method according to the invention, the method comprises measuring the level of antibodies in a biological sample specific for a UL42 peptide comprising, or consisting of SEQ ID NO 003 (UL42 393 - 448). In certain embodiments of the method according to the invention, the method comprises measuring the level of antibodies in a biological sample specific for a UL42 peptide comprising, or consisting of SEQ ID NO 004 (UL42 421 - 476). In certain embodiments of the method according to the invention, the method comprises measuring the level of antibodies in a biological sample specific for a UL42 peptide comprising, or consisting of SEQ ID NO 005 (UL42 449 - 488).

In certain embodiments of the method according to the invention, the method comprises measuring the level of antibodies in a biological sample specific for a variant UL42 peptide of use as a capture antigen, having at most 1 amino acid substitution, insertion, or deletion for every 10 amino acids in length compared to a peptide selected from the list consisting of SEQ ID NO 003, SEQ ID NO 004, and SEQ ID NO 005. In certain embodiments of the method according to the invention, the method comprises measuring the level of antibodies in a biological sample specific for 2 peptides selected from the list consisting of SEQ ID NO 3, SEQ ID NO 004, and SEQ ID NO 005. In particular embodiments of the method according to the invention, the method comprises measuring the level of antibodies in a biological sample specific for all of the peptides SEQ ID NO 3, SEQ ID NO 004, and SEQ ID NO 005.

In certain embodiments of the method according to the invention, the method comprises measuring the level of antibodies specific for a UL48 region polypeptide comprising SEQ ID NO 002. In other embodiments, the method to measure HSV-specific antibodies comprises measuring the level of antibodies specific for a variant UL42 region polypeptide comprising a polypeptide having up to 5 amino acids substitutions compared to SEQ ID NO 002.

In certain embodiments of the method according to the invention, the method comprises measuring the level of antibodies in a biological sample specific for a UL48 peptide comprising the sequence SEQ ID NO 006 (UL48 337 - 392). In other embodiments, the UL48 peptide consists of SEQ ID NO 006.

In certain embodiments of the method according to the invention, it comprises measuring the level of antibodies in a biological sample specific for a UL48 peptide comprising the sequence SEQ ID NO 007 (UL48 365 - 420). In other embodiments, the UL48 peptide consists of SEQ ID NO 007.

In certain embodiments of the method according to the invention, the method comprises measuring the level of antibodies in a biological sample specific for UL48 peptides SEQ ID NO 006, or 007 using a variant UL48 peptide having at most 1 amino acid substitution, insertion, or deletion for every 10 amino acids in length compared to a peptide selected from the list consisting of SEQ ID NO 006, and SEQ ID NO 007.

In particular embodiments of the method according to the invention, the method comprises measuring the level of antibodies in a biological sample specific for both peptides SEQ ID NO 006, and SEQ ID NO 007.

In particular embodiments, the method comprises measuring the level of antibodies specific for both non-variant regions SEQ ID NO 001, and SEQ ID NO 002.

In particular embodiments, the method comprises measuring the level of antibodies specific for at least one peptide selected from the list consisting of SEQ ID NO 003, 004 and 005, and at least one peptide selected from the list consisting of SEQ ID NO 006 and 007.

In particular embodiments, the method comprises measuring the level of antibodies specific for all 5 non-variant peptides SEQ ID NO 003, 004, 005, 006, and 007.

In certain embodiments, the HSV antigen is a conjugate antigen, wherein two or more of the HSV polypeptides and peptides described in this section are linked to form a single molecule, for example by means of a peptide linker forming a single polypeptide comprising multiple HSV antigens.

In certain embodiments of the method according to the invention, the level of antibodies bound to the at least one HSV antigen is determined by contacting the sample with a molecular probe specific for human immunoglobulin (Ig), said molecular probe being conjugated to a detectable label.

In particular embodiments, the level of antibodies specific for the HSV antigen, or HSV antigens is detected by means of an anti-IgG-antibody conjugated to a detectable label. In some embodiments, the molecular probe specific for human Ig is an anti-IgG antibody.

In some embodiments, the method comprises a step wherein the level of the at least one antibody detected in the sample, is compared to a threshold.

In some embodiments, the method comprises a step wherein the level of the at least one antibody detected in the sample, is compared to a negative control sample.

In certain embodiments of the method according to the invention, the level of HSV antigen-specific antibodies in the biological sample is measured by means of a passive agglutination assay. In certain embodiments, the level of HSV antigen-specific antibodies in the biological sample is detected by a hemagglutination assay. In certain embodiments, the level of HSV antigen-specific antibodies in the biological sample is detected by western blot. In certain embodiments, the level of HSV antigen-specific antibodies in the biological sample is detected using an enzyme-linked immunosorbent assay (ELISA). In certain embodiments, the level of HSV antigen-specific antibodies in the biological sample is measured using a lateral flow device. In certain embodiments, the level of HSV antigen-specific antibodies in the biological sample is detected using a fluorescence immunoassay. In certain embodiments, the level of HSV antigen-specific antibodies in the biological sample is measured using a radioimmunoassay. In certain embodiments, the level of at least two HSV antigen-specific antibodies in the biological sample is detected by multiplexed flow cytometry immunoassay.

In certain embodiments of the method according to the invention an ELISA methodology is used, which has the advantage of providing quantitative antibody level measurements. An indirect ELISA is most commonly used to detect antigen-specific antibodies. The principle of this assay is to coat a solid phase carrier with HSV polypeptides or peptides. This binds with serum antibody, and an enzyme-linked antibody to produce a chromogenic reaction which reveals a precise level of specific antibody in the sample, compared to a control standard, or positive and negative controls.

In certain embodiments, the method comprises contacting the biological sample with a plate well coated with at least one antigen as specified according any one of the claims 1 to 7 (i.e. the antigen is bound to, or otherwise immobilized on the plate). Optionally removal of unbound proteins from the plate well is performed in a washing step. The method comprises contacting the plate well with a molecular probe specific for human immunoglobulin (Ig), said molecular probe being conjugated to a detectable label, for example an enzyme which enables a chromogenic or chemiluminescent reaction with a substrate.

In certain embodiments, the level of at least two HSV antigen-specific antibodies in the biological sample is detected by flow cytometry-based immunoassay, for example a cytometric bead array wherein each antigen is conjugated to a differentiated beads conjugated with varying size, and/or levels of a fluorescent marker. Differentiated beads can be coated with HSV antigens according to the invention to provide information regarding which HSV epitope is targeted by patient antibodies.

In some embodiments of the method according to the invention, the method delivers a qualitative point of care result by measuring the level of HSV-antigen specific antibodies in a biological sample. The method may comprise contacting a biological sample HSV antigens according to the invention, for example HSV1 antigens SEQ ID NO 001 (or peptides SEQ ID NO 003, 004, 005), and SEQ ID NO 002 (or peptides SEQ ID NO 006 and 007) fixed on a nitrocellulose membrane, in a contacting step. The sample is contacted with an antibody-specific detection probe, for example, gold-nanoparticle conjugated anti-Ig, forming the Ag-Ab-Ab positive signal when the sample comprises antibody specific for any one of the HSV antigens fixed to the membrane.

In certain embodiments, the level of HSV antigen-specific antibodies in the biological sample is measured using a lateral flow immunochemical method in a lateral flow device kit. A lateral flow device kit typically comprises a sample pad, a target (HSV antigen) conjugate release pad, a nitrocellulose membrane with immobilised anti-Ig antibodies on its surface, an absorbent pad in plastic backing, and biological reagents. In some embodiments of the method, the patient sample is contacted with at least one HSV antigen conjugated to a detectable label at a conjugate release pad. In some embodiments, the detectable label is a nanoparticle (e.g. colloidal gold). In the presence of HSV-specific antibody in the patient sample, a complex forms with the HSV antigen in the reagent mix, which is then immobilised by the anti-Ig antibodies to become a visible line in a positive section of the test cartridge. Optionally a positive control line is also included, such that if no such antibody is present, HSV-antigen is instead immobilised by anti-HSV antibodies to form a visible line in a control region of the nitrocellulose membrane.

*Biological samples*

The method as described in the preceding section entitled *Method to detect HSV1 specific antibodies* is designed for biological samples obtained from a patient whose symptoms and the result of a viral detection method indicate a diagnosis of herpes simplex virus encephalitis (HSE). Sample acquisition from the patient is not a part of the method.

The sample to which the method is applied is not particularly limited, but relates to measurement of antibodies. Liquid samples such as blood, serum or plasma representative of systemic antibodies may be one informative input, and the inventors also identify a predictive relationship for cerebrospinal fluid antibody levels. The method can be applied to experimental samples, such as tissue homogenates.

The term HSE in the context of the specification refers to encephalitis, inflammation of the brain associated with HSV infection. Encephalitis can be caused by inflammation directed at infectious agents, such as viruses, or autoimmunity. Symptoms include confusion, personality changes, seizures, problems with movement, loss of consciousness, or changes in sight or hearing. In infants, symptoms may include bulges or protrusions of the skull, nausea, stiffness, reduced appetite. Viral encephalitis associated with HSV infection is often additionally associated with flu-like symptoms such as headache, fever, aches, and fatigue.

In addition to encephalitis symptoms, diagnosis of HSE is also based on confirmation of viral infection by a viral detection method. A HSV infection can be confirmed via standard laboratory tests available to clinicians which can detect whole virus, viral proteins or glycoproteins, genetic material, or anti-HSV antibody titers. These include point of care microfluidics-based antigen testing, or nucleic acid amplification of viral genes by polymerase chain reaction or loop-mediated isothermal amplification.

In certain embodiments of the method according to any aspect of the invention, the biological sample is a blood sample.

In certain embodiments of the method according to any aspect of the invention, the biological sample is a plasma sample.

In certain embodiments of the method according to any aspect of the invention, the biological sample is a serum sample.

In certain embodiments of the method according to any aspect of the invention, the biological sample is a cerebral spinal fluid sample.

In certain embodiments of the method according to any aspect of the invention, the method is performed with a sample obtained from a patient who experienced HSV encephalitis symptom onset at least two weeks before the biological sample was obtained.

*Method to predict the prognosis* of *an HSE patient*

A next aspect of the invention is a method to predict the prognosis of a patient diagnosed with herpes simplex virus encephalitis. The method comprising the steps of performing the method as specified in any one of the claims 1 to 11, according to the methods outlined in the section *Methods to detect HSV1-specific antibodies.* The input is a sample obtained from the patient as described in the section *Biological samples.*

The method to predict HSE patient prognosis further comprises assigning the patient:

- a high likelihood of developing HSV-induced NMDAR encephalitis if the level of antibody specific for the at least one HSV antigen detected in the sample is equal to or above a threshold; or
- a low likelihood of developing HSV-induced anti-NMDAR encephalitis if the level of antibody specific for the at least one HSV antigen is below said threshold.

In some embodiments, the method to predict HSE patient prognosis further comprises assigning the patient:

- a high likelihood of negative clinical outcomes associated with NMDAR encephalitis, if the level of antibody specific for the at least one HSV antigen detected in the sample is equal to or above a threshold; or
- a low likelihood of negative clinical outcomes associated with NMDAR encephalitis, if the level of antibody specific for the at least one HSV antigen is below said threshold.

If the level of antibody specific for more than one HSV antigen is measured, a low likelihood of developing HSV-induced anti-NMDAR encephalitis (and/or associated poor clinical outcomes), is assigned to the subject, if the level of antibody specific for all HSV antigens tested is below said threshold, as all HSV antigens according to the invention were associated with eventual NMDAR encephalitis onset in a subset of patients.

The skilled person may establish thresholds that indicate that antibodies specific for HSV-antigens according to the invention (see *Methods to detect HSV1-specific antibodies*) which are appropriate for the measurement methodology being used.

In some embodiments, the threshold is a numerical threshold established to indicate background levels of antibody binding to the HSV antigens used in the method, as present in a cohort of HSE patients who did not develop NMDAR encephalitis within 2 years.

In some embodiments, the threshold is a numerical threshold established which is representative of a positive control sample, for example the average level of antibodies specific for the HSV antigens in samples obtained from a cohort of HSE patients who went on to develop NMDARE.

In some embodiments, the threshold is based on the level of antibody binding in negative control sample comprising antibody that is specific to an irrelevant, non-HSV antigen.

In some embodiments, the threshold is based on the level of antibody binding in positive control sample comprising antibody that is specific to HSV antigens according to the invention.

In some embodiments of the method to predict the prognosis of an HSE patient according to the invention, the sample is a serum sample, and a high likelihood of developing NMDAR encephalitis is defined as a more than 80% probability likelihood of developing NMDAR encephalitis in the next 2 years, and a low likelihood of developing NMDAR encephalitis is defined as a less than 5% chance of developing NMDAR encephalitis in the next 2 years.

Most patients develop NMDAR encephalitis within 3 months of experiencing HSE. In some embodiments of the method to predict the prognosis of an HSE patient according to the invention, the sample is a serum sample, and a high likelihood of developing NMDAR encephalitis is defined as a more than 80% probability likelihood of developing NMDAR encephalitis in the next 3 months, and a low likelihood of developing NMDAR encephalitis is defined as a less than 5% chance of developing NMDAR encephalitis in the next 3 months.

*Immunomodulatory agents*

Another aspect of the invention relates to immunomodulatory agents for use in a patient assigned a high-risk of developing NMDAR encephalitis according to the methods described above (*Method to predict the prognosis of an HSE patient*). A clinician may choose to administer such immunomodulatory agents based on the presence of HSV-antigen specific antibodies associated with NMDAR encephalitis, depending on the context of the patient's clinical course, and present condition. Signature HSV antibodies can be detected earlier than symptoms of encephalitis arise, and provide a promising avenue to apply the current gold standard therapeutic agents to delay, reduce, or prevent antibody-driven pathology.

In particular embodiments, the immunomodulatory agent is for use in a patient assigned a high-risk of developing NMDAR encephalitis, and further displaying et least one symptom of neurological inflammation such as confusion, personality changes, seizures, problems with movement, or alterations of consciousness.

In certain embodiments, the immunomodulatory agent is selected from the list consisting of an immunosuppressive agent or an intravenous immunoglobulin formulated for intravenous infusion (IVIG).

Another aspect of the invention is a combination of IVIG and an immunosuppressive agent for use in a patient assigned a high-risk of developing NMDARE.

Another aspect of the invention is a method of treating a patient assigned a high likelihood of developing NMDAR encephalitis according to the method of any one of the claims 12 to 15 (see *Method to predict the prognosis of an HSE patient*). The method comprises administering the patient an effective dose of an immunomodulatory agent selected from an immunosuppressive agent or an intravenous immunoglobulin infusion (IVIG), and/or performing plasmapheresis. In particular embodiments, the current gold standard first line treatment for autoimmune encephalitis is applied to the patient comprising administering corticosteroids in conjunction with IVIG and/or plasmapheresis.

In certain embodiments, the method of treating a patient further comprises measuring the level of NMSDAR-specific antibodies present in a sample obtained from the patient.

In certain embodiments, the immunomodulatory agent is a corticosteroid (intravenous or oral formulated). In particular embodiments, the immunomodulatory agent is prednisone, or methyl prednisone.

In certain embodiments, the immunomodulatory agent is azathioprine. In certain embodiments, the immunomodulatory agent is mycophenolate mofetil. In certain embodiments, the immunomodulatory agent is azathioprine. In certain embodiments, the immunomodulatory agent is cyclophosphamide. In certain embodiments, the immunomodulatory agent is bortezomib. In certain embodiments, the immunomodulatory agent is methotrexate.

In certain embodiments, the immunomodulatory agent is a B cell-depleting monoclonal antibody. In certain embodiments, the immunomodulatory agent is inebilizumab. In certain embodiments, the immunomodulatory agent is uplizna. In certain embodiments, the immunomodulatory agent is rituximab. In certain embodiments, the immunomodulatory agent is alemtuzumab.

In certain embodiments, the immunomodulatory agent is a monoclonal antibody against interleukin-6 receptor. In certain embodiments, the immunomodulatory agent is tocilizumab.

In certain embodiments, the immunomodulatory agent is an anti-rheumatic drug (a class of drugs sometimes referred to as disease-modifying antirheumatic drugs, or DMARDS). In some embodiments, the immunomodulatory agent is methotrexate, sulfasalazine, hydroxychloroquine, leflunomide, or azathioprine. In some embodiments, the immunomodulatory agent is abatacept, adalimumab, anakinra, certolizumab pegol, etanercept, golimumab, sarilumab, secukinumab, tocilizumab, or ustekinumab.

*Further aspects of the invention*

Another aspect of the invention is a kit for HSE biological samples comprising any one of the HSV antigens SEQ ID NO 001-007, or variants thereof described above, and optionally, a molecular probe specific for human immunoglobulin (Ig), wherein said molecular probe is conjugated to a detectable label. The kit may optionally also include positive/negative controls. The kit may optionally include a standard comprising a known concentrations of human IgG binding specifically to at least one HSV antigen according to the invention.

Another aspect of the invention is an HSV antigen as defined by any one of the claims 1 to 7, for the manufacture of a kit for treating NMADRE.

Another aspect of the invention is an immunomodulatory agent for the manufacture of a kit for treating NMADRE.

Another aspect of the invention is a kit comprising an ELISA assay well-plate, and a reagent comprising an HSV polypeptide or polypeptide according to the invention, and a reagent comprising an anti Ig detection antibody conjugated to a chromogenic label.

Another aspect of the invention is a lateral flow kit comprising a test cartridge comprising a sample pad, a conjugate release pad comprising an HSV antigen according to the invention, a nitrocellulose membrane with immobilised anti-Ig antibodies on its surface, and an absorbent pad in plastic backing. The test cartridge optionally comprises a blood separation membrane which removes red blood cells from sample applied to the device.

*Pharmaceutical Compositions, Administration/Dosage Forms and Salts*

According to one aspect of the compound according to the invention, the compound according to the invention is provided as a pharmaceutical composition, pharmaceutical administration form, or pharmaceutical dosage form, said pharmaceutical composition, pharmaceutical administration form, or pharmaceutical dosage form comprising at least one of the compounds of the present invention or a pharmaceutically acceptable salt thereof and at least one pharmaceutically acceptable carrier, diluent or excipient.

The skilled person is aware that any specifically mentioned drug compound mentioned herein may be present as a pharmaceutically acceptable salt of said drug. Pharmaceutically acceptable salts comprise the ionized drug and an oppositely charged counterion. Non-limiting examples of pharmaceutically acceptable anionic salt forms include acetate, benzoate, besylate, bitartrate, bromide, carbonate, chloride, citrate, edetate, edisylate, embonate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate, phosphate, diphosphate, salicylate, disalicylate, stearate, succinate, sulfate, tartrate, tosylate, triethiodide and valerate. Non-limiting examples of pharmaceutically acceptable cationic salt forms include aluminum, benzathine, calcium, ethylene diamine, lysine, magnesium, meglumine, potassium, procaine, sodium, tromethamine and zinc.

In certain embodiments of the invention, the compound of the present invention is typically formulated into pharmaceutical dosage forms to provide an easily controllable dosage of the drug and to give the patient an elegant and easily handled product.

Similarly, a dosage form for the prevention or treatment of a patient assigned a high risk of developing NMDAR encephalitis is provided, comprising an immunomodulatory agent or immunosuppressive drug molecule according to any of the above aspects or embodiments of the invention.

The invention further encompasses a pharmaceutical composition comprising a compound of the present invention, or a pharmaceutically acceptable salt thereof, and a pharmaceutically acceptable carrier. In further embodiments, the composition comprises at least two pharmaceutically acceptable carriers, such as those described herein.

Certain embodiments of the invention relate to a dosage form for parenteral administration, such as subcutaneous, intravenous, intra-hepatic or intramuscular injection forms. Optionally, a pharmaceutically acceptable carrier and/or excipient may be present.

The dosage regimen for the compounds of the present invention will vary depending upon known factors, such as the pharmacodynamic characteristics of the agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the compounds of the invention may be administered in a single daily dose, or the total daily dosage may be administered in divided doses of two, three, or four times daily.

In certain embodiments, the pharmaceutical composition or combination of the present invention can be in unit dosage of about 1-1000 mg of active ingredient(s) for a subject of about 50-70 kg. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

Present clinical recommendations for therapeutic agents applied to autoimmune encephalitis patients such as those identified by the methods herein are known to the skilled person. Specific dosages, combinations and treatment regimens have been developed to specifically treat anti-NMDAR encephalitis (Samanta & Lui, 2024).

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers, and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

## Method of Treatment according to the invention

The invention further encompasses, as an additional aspect, the use of an immunomodulatory agent such as an immunosuppressive drug, or IVIG as identified herein, for use in a method of manufacture of a medicament for the treatment or prevention of NMDAR encephalitis.

Similarly, the invention encompasses methods of treatment of NMDAR encephalitis, comprising administering to a patient in need thereof a therapeutically effective amount of an immunomodulatory agent such as an immunosuppressive drug, or a human IgG formulated for intravenous use, as specified in detail herein.

Another aspect of the invention relates to a method of treating an HSE patient comprising obtaining a biological sample comprising antibodies, performing the method to detect HSV-antibodies as laid out in claims 1 to 11, and performing plasmapheresis if the level of antibodies specific for HSV antigens is above a threshold.

The invention further encompasses the use of at least one of the HSV antigens identified herein, particularly a polypeptide comprising at least one sequence selected from SEQ ID NO 001-005, for use in the manufacture of a kit for the detection of NMDAR encephalitis, or for identifying patients at high risk of developing NMDAR encephalitis in the future after an HSV encephalitis episode.

Another aspect of the invention relates to a biomarker composition comprising an HSV antigen according to the invention. The invention encompasses a method of providing information for predicting whether an HSE patient will develop NMDARE comprising the steps of contacting a patient sample with HSV antigens as specified by claims 1 to 11, or items A to K listed below.

Wherever alternatives for single separable features are laid out herein as "embodiments," it is to be understood that such alternatives may be combined freely to form discrete embodiments of the invention disclosed herein. Thus, any of the alternative embodiments for an HSV antigen may be combined with any of the alternative embodiments of a diagnostic, or a prognostic method, or a method of treating a patient, and these combinations may be combined with any feature mentioned herein.

The invention further encompasses the following items relating to UL42 and UL48 polypeptides of use for methods to detect HSV-specific antibodies of prognostic value in classifying HSE patients according to whether they are likely to develop NMDARE by analysis of patient samples.

A. A method to detect Herpes simplex virus Type 1 (HSV1)-specific antibodies, the method comprising measuring a level of antibodies specific for at least one HSV antigen in a biological sample, characterized in that the HSV antigen is selected from the list consisting of:

- a UL42 region polypeptide comprising, or consisting of SEQ ID NO 001, or a variant UL42 region polypeptide comprising, or consisting of a polypeptide having at most 1 amino acid substitution, insertion, or deletion for every 10 amino acids in length compared to SEQ ID NO 001; or
- at least one UL42 peptide comprising, or consisting of 5 to 80 consecutive amino acids comprised in SEQ ID NO 001, and/or a variant UL42 peptide comprising, or consisting of a of 5 to 80 consecutive amino acids, an having at most 1 amino acid substitution, insertion, or deletion for every 10 amino acids in length compared to said UL42 peptide;
  and/or
- a UL48 region polypeptide comprising, or consisting of SEQ ID NO 002, or a variant UL48 region polypeptide comprising, or consisting of a polypeptide having at most amino acid substitution, insertion, or deletion for every 10 amino acids in length compared to SEQ ID NO 002; or

- at least one UL48 peptide comprising, or consisting of 5 to 80 consecutive amino acids comprised in SEQ ID NO 002; and/or a variant UL48 peptide comprising, or consisting of 5 to 80 consecutive amino acids, and having at most amino acid substitution, insertion, or deletion for every 10 amino acids in length compared to said UL48 peptide.

B. The method according to item A, wherein the method comprises measuring the level of antibodies specific for the UL42 region polypeptide, or the variant UL42 region polypeptide as specified in item A.

C. The method according to item A, wherein:

- the UL42 peptide comprises, or consists of a peptide selected from the list consisting of SEQ ID NO 003, SEQ ID NO 004, and SEQ ID NO 005; or
- the variant UL42 peptide comprises, or consists of a peptide having at most 1 amino acid substitution, insertion, or deletion for every 10 amino acids in length compared to a peptide selected from the list consisting of SEQ ID NO 003, SEQ ID NO 004, and SEQ ID NO 005.

D. The method according to item A or C, wherein the method comprises measuring the level of antibodies specific for the peptides SEQ ID NO 3, SEQ ID NO 004, and SEQ ID NO 005.

E. The method according to any one of the items A to D, wherein the method comprises measuring the level of antibodies specific for the UL48 region polypeptide, or the variant UL48 region polypeptide as specified in item A.

F. The method according to any one of the items A to D, wherein:

- the UL48 peptide comprises, or consists of a peptide selected from the list consisting of SEQ ID NO 006, and SEQ ID NO 007; and/or
- the variant UL48 peptide comprises, or consists of a peptide having at most 1 amino acid substitution, insertion, or deletion for every 10 amino acids in length compared to a peptide selected from the list consisting of SEQ ID NO 006, and SEQ ID NO 007.

G. The method according to any one of the items A to D, or F, wherein the method comprises measuring the level of antibodies specific for the peptides SEQ ID NO 006, and SEQ ID NO 007.

H. The method according to any one of the previous items, wherein the level of antibodies bound to the at least one HSV antigen is determined by contacting the sample with a molecular probe specific for human immunoglobulin (Ig), said molecular probe being conjugated to a detectable label.

I. The method according to any one of the preceding items, wherein method of measuring the level of antibodies comprises a detection methodology selected from the list consisting of passive agglutination, hemagglutination, western blot, enzyme-linked immunosorbent assay (ELISA), lateral flow device assay, fluorescence immunoassay, multiplexed flow cytometry immunoassay, luciferase immunoprecipitation assay, and radioimmunoassay

J. The method according to any one of the preceding items, wherein the biological sample is a blood sample, particularly, wherein the biological sample is a plasma sample or a serum sample.

K. The method according to any one of the items A to I, wherein the biological sample is a cerebral spinal fluid sample.

L. A method to predict the prognosis of a patient diagnosed with herpes simplex virus encephalitis comprising the steps of:

- performing the method as specified in any one of the claims 1 to 11 on a biological sample obtained from a patient;
- assigning the patient:

  • a high likelihood of developing anti-N-methyl-D-aspartate receptor (NMDAR) encephalitis if the level of antibody specific for the at least one HSV antigen detected in the sample is equal to or above a threshold; or
  • a low likelihood of developing anti-NMDAR encephalitis if the level of antibody specific for the at least one HSV antigen is below said threshold.

M. The method according to item L, wherein the sample is a serum sample, and a high likelihood of developing NMDAR encephalitis is defined as a more than 80% probability of developing NMDAR encephalitis in the next 2 years, and a low likelihood of developing NMDAR encephalitis is defined as a less than 5% probability of developing NMDAR encephalitis in the next 2 years.

N. The method according to any one of the items L or M, wherein the patient experienced HSV encephalitis symptom onset at least two weeks before the biological sample was obtained.

O. An immunomodulatory agent selected from the list consisting from an immunosuppressive agent or intravenous immunoglobulin, for use in a patient assigned a high likelihood of developing NMDAR encephalitis according to the method of any one of the items L to N, particularly wherein the immunomodulatory agent is an immunosuppressive agent selected from the list consisting of a corticosteroid, azathioprine, mycophenolate mofetil, azathioprine, bortezomib, cyclophosphamide, methotrexate, rituximab, alemtuzumab, and/or tocilizumab.

The invention is further illustrated by the following examples and figures, from which further embodiments and advantages can be drawn. These examples are meant to illustrate the invention but not to limit its scope.

_Description of the Figures_

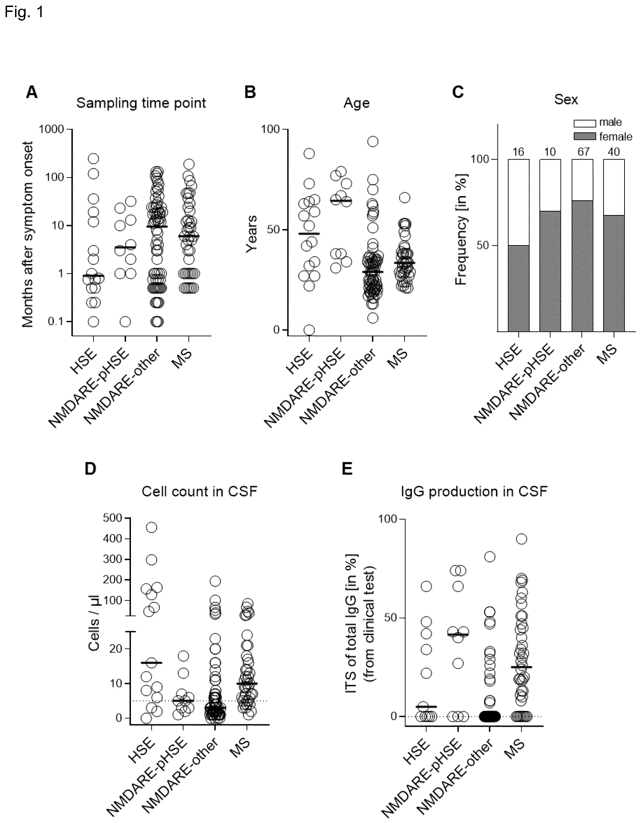

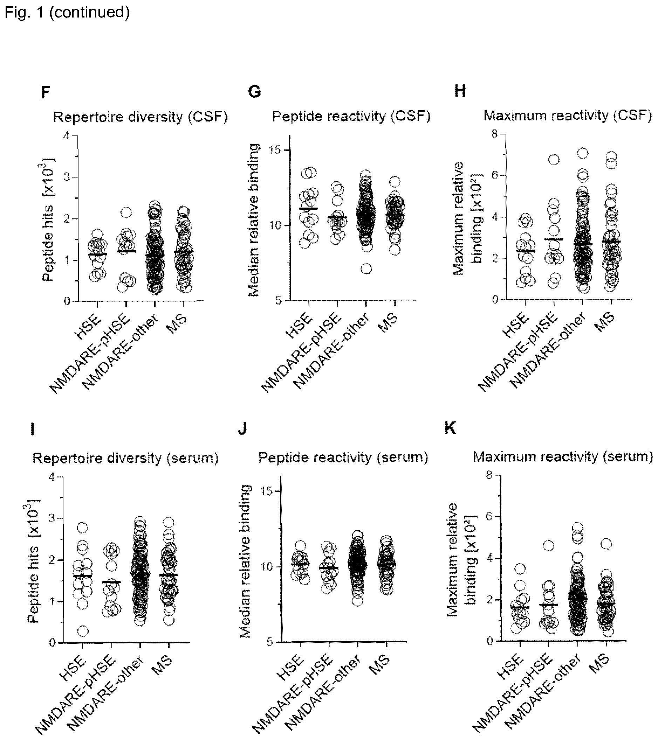

Fig. 1 shows patient cohorts and global characteristics of antibody repertoires. (A-E) Patient characteristics are shown by clinical cohorts for (A) sampling time point after symptom onset, (B) age, (C) sex distribution, (D) cell count in CSF and (E) ITS of total IgG from clinical testing, i.e. the fraction of the IgG produced in the CSF over the total CSF IgG (clinical ITS of total IgG). Data are from 133 individuals, shown from earliest sampling time point if multiple were available and with three individuals occurring in HSE and NMDARE-pHSE cohorts. Bars indicate median. Dotted lines indicate threshold for positivity. Number above bars in C indicate sample size. For comparison in (A), a Kruskal-Wallis test with post-hoc multiple comparison using Dunn's test was used and revealed no significant differences. (F-K) Scattered dot plots display by each cohort (F, I) repertoire diversities (measured as count of peptide reactivities that fulfil hit criteria, see Methods), (G, J) median and (H, K) maximum relative binding of all peptide hits for (F-H) CSF and (I-K) serum antibodies. Data are from 148 paired CSF/serum samples derived from 128 individuals. Bars indicate mean. For comparison one-way ANOVA tests with post-hoc Tukey's multiple comparison were used and revealed no significant differences.

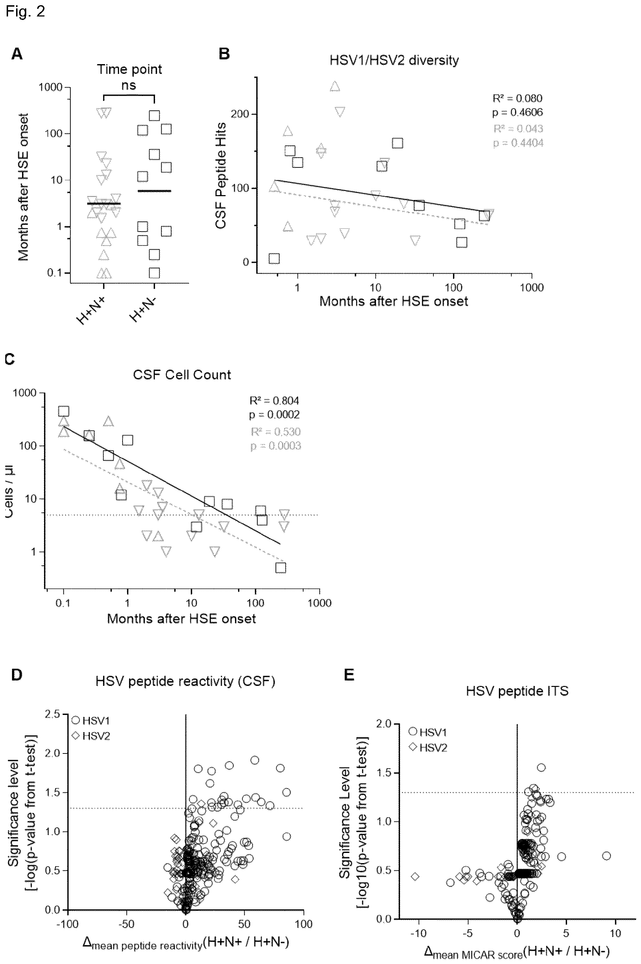

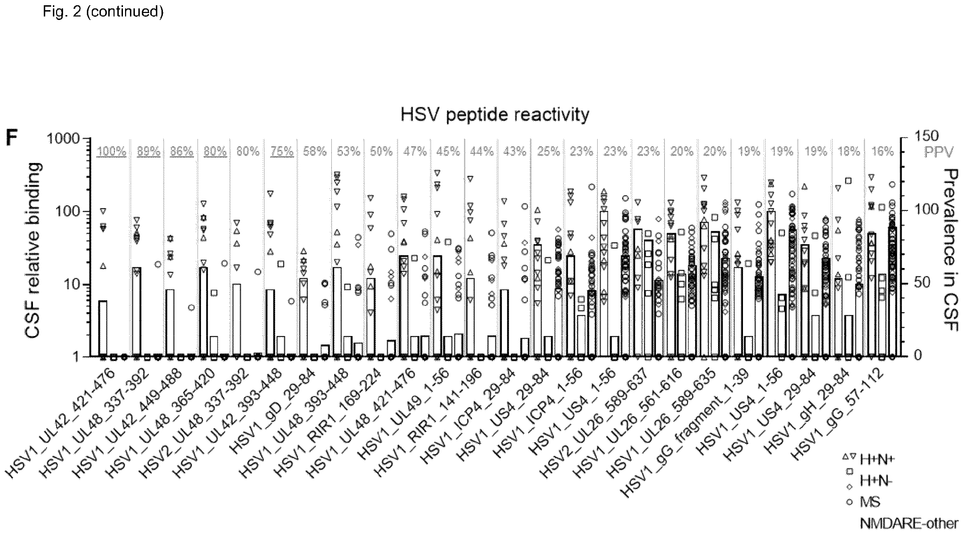

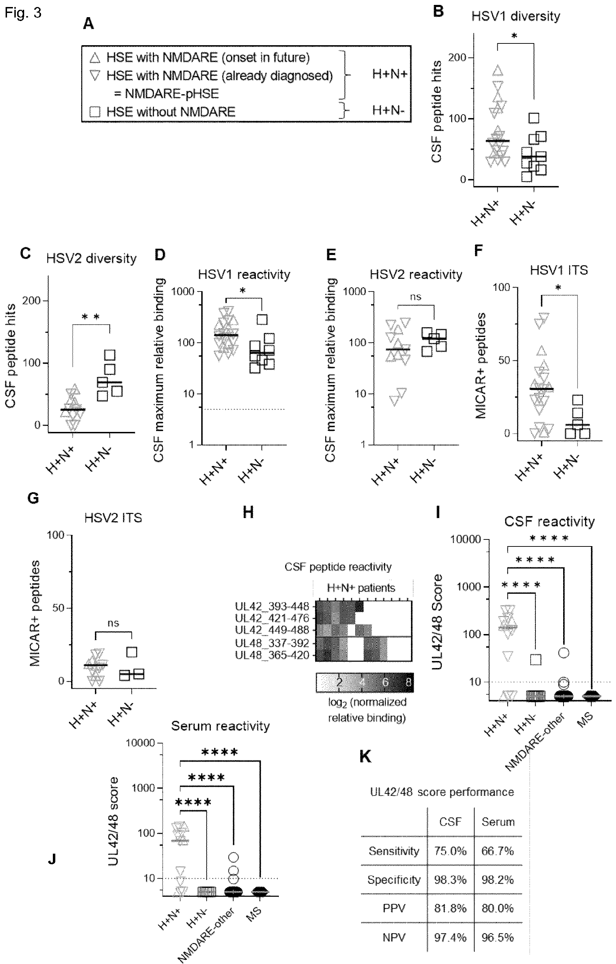

Fig. 2 shows comparison HSV responses within non-HSE patients and within HSE subgroups. (A) Scattered dot plots display time points from 21 H+N+ samples (13 individuals) and 11 H+N-samples (10 individuals). Upward-point arrowheads represent H+N+ samples before NMDARE was diagnosed, downward-point arrowheads represent after NMDARE manifestation. For comparison, a Mann-Whitney test was used, ns = not significant. (B) Correlations of CSF antibody diversities to herpes simplex viruses (HSV1 and HSV2 combined) with sampling time points were determined using linear regression models, separately for H+N+ and H+N- groups. Symbols as shown above and samples as in A, with those excluded that were taken less than two weeks after HSE onset. (C) Correlations of CSF cell count (as sign of inflammatory CSF) with sampling time points were determined using linear regression models, separately for H+N+ and H+N- groups. Symbols and samples as in A, with including those that were taken less than two weeks after HSE onset. Dotted line indicate threshold for positivity. (D) Differential reactivities are shown for 305 HSV1 and HSV2 peptides (shown if detected in at least one H+N+ or H+N- individual). Two-tailed t-tests were used to compare peptide reactivities between H+N+ (n= 12) and H+N- (n = 8) individuals. Earliest sampling time points were analyzed if multiple were available. Symbols as in A. Positive $\Delta$means indicate stronger reactivity in H+N+ group. HSV1 (circles) and HSV2 (diamonds). Dotted lines indicate threshold for statistical significance. (E) Differential degree of ITS are shown for 305 HSV1 and HSV2 peptides (as in J). Two-tailed t-tests were used to compare MICAR peptide scores between H+N+ (n= 12) and H+N- (n = 5) individuals with available paired serum. Symbols as in A. Positive $\Delta$means indicate enhanced ITS in H+N+ group. Dotted lines indicate threshold for statistical significance. (F) Scattered dot plots display CSF antibody peptide reactivities against 24 HSV peptides (selected if significantly higher reactivities or MICAR scores in H+N+ vs. H+N- patients, see D, E). Symbols represent individual relative binding values (left axis), bars show prevalence in CSF by group (right axis) and grey numbers indicate PPVs (top). For five HSV1 signature peptides (with PPV ≥ 75%) PPV values are underlined (see also Figures 3H-K). Data are from 82 CSF samples (82 individuals), shown from earliest time point if multiple were available (in H+N+ group from first time point after NMDARE manifestation) and if an antibody response to the respective virus is prevalent in CSF or serum, as defined by VAR scores >0.72 (see Methods).

Fig. 3 shows enhanced HSV antibody response to distinct peptides is associated with NMDARE development. (A) Illustration of H+N+ and H+N- group composition. (B-G) Scattered dot plots display CSF antibody (B-C) diversity, (D-E) reactivity and (F-G) ITS for HSV1 and HSV2 antibody reactivities as indicated. Data are from 18 H+N+ (n = 12 individuals) and 9 H+N- samples (n = 8 individuals), with samples excluded if taken less than two weeks after HSE onset. Symbols as in A. Bars indicate median. For comparison, Mann-Whitney tests were used, ns = not significant, * p < 0.05, ** p < 0.01. Reactivity in D-E is measured as mean of highest five relative binding values from five peptides representing five proteins of the respective virus, shown if antibody response to the respective virus is prevalent in CSF or serum (defined by VAR scores >0.72, see Methods). ITS in F-G is measured as count of MICAR+ peptides representing the respective virus. Dotted lines indicate threshold for positivity. (H) A heat map displays CSF antibody reactivities to five signature HSV1 peptides for H+N+ individuals (from first time point after NMDARE manifestation). See also Figure 2L for identification of signature peptides. (I-J) Scattered dot plots display UL42/48 scores for (I) CSF and (J) serum antibody reactivities. UL42/48 scores are aggregated relative binding values to five signature peptides (as in H). Data are from 133 CSF / 128 serum samples from 133 individuals, excluded in H+N+/H+N- groups if less than two weeks after HSE onset. Symbols as in A. Bars indicate median. For comparison, Kruskal-Wallis tests were used with post-hoc multiple comparison using Dunn's test, **** $p < 0.0001$ or not significant if not shown. Dotted lines indicate threshold of positivity. (K) Parameters of UL42/48 score test performance are shown (samples and data as in I-J. NPV = negative predictive value, PPV = positive predictive value.

Examples

*Example 1: Patient group CSF and serum antibody repertoires*

The presence of antibodies in any body fluid compartment can result from passive antibody diffusion from blood or from local production. The latter is indicative of a compartment-specific immune response with disease relevance and can be identified by antibody indices as ratios of quantitative antibody measurements from the compartment of interest and blood. This paradigm has been used for decades, especially in neurology, where antibody indices provide evidence of antibody production within the central nervous system (CNS), referred to as intrathecal antibody synthesis (ITS). ITS can be quantified for total or for antigen specific antibodies and is a characteristic feature for a variety of neurological disorders. In a multicenter study, the inventors collected 148 pairs of CSF and serum samples from 133 patients with MS, HSE, and NMDARE. The NMDARE cohort was subdivided into patients with preceding HSE (NMDARE-pHSE) and those without (NMDARE-other). In all four groups, sample collection was at similar intervals after symptom onset. Patient data and routine laboratory tests correspond with disease-typical features in age, gender, CSF cell count and ITS of total immunoglobulin G (IgG) (Fig. 1A-E)).

The inventors performed PhIP-Seq measurements using five phage libraries to comprehensively profile the IgG antibody binding reactivity against nearly 600,000 peptides including human viruses (VirScan). Across the disease groups, the antibody repertoires were similar in numbers of peptide reactivities, median strength of reactivity and maximum strength of reactivity (Figures 1F-K), assuring comparability of these results. This pattern was also observed when excluding those CSF samples with less than two $\mu$g IgG input. CSF-specific hits correlated with ITS of total IgG as determined by routine clinical testing, i.e. the fraction (in %) of the IgG produced in the CSF among the total IgG in CSF ("clinical ITS of total IgG"), demonstrating that PhIP-Seq has the potential to identify CNS compartment-specific immune responses.

*Example 2: Enhanced HSV antibody response to distinct peptides is associated with NMDARE development*

HSV antibody responses were dissected for characteristics that associate with the secondary development of NMDARE after HSE. In the study cohorts, HSV antibodies were prevalent in serum and CSF of all HSE and NMDARE-pHSE patients, except for individuals who experienced symptom onset within two weeks before sampling (and thus before maturation of an antibody response).

The inventors next looked for differences in the HSV response in HSE patients with NMDARE (H+N+) versus those that did not develop NMDARE (H+N-) and re-grouped the HSE and NMDARE-pHSE patients accordingly (Figure 3A). Both groups contained similar distributions of sampling time points (Figure 2A) with continued detection of HSV antibodies even years after acute signs of inflammation have vanished (Figures 2B-C). H+N+ patients display enhanced antibody responses towards HSV1, measured in increased diversity of targeted peptides, elevated binding strength and increased counts of MICAR+ peptides, none of which was observed for HSV2 (Figures 3B-G).

The inventors identified 22 HSV1 and two HSV2 peptides that are more reactive and/or have higher MICAR scores in H+N+ patients but none that are specific for H+N- (Figures 2D, 2E). Of these HSV1 peptide reactivities, five "signature" peptides are not only specific for H+N+ when compared to H+N-, but also rarely detected in MS patients and NMDARE-other patients, resulting in positive predictive values (PPV) within the overall study cohort between 75 and 100% (Figure 2F). These five peptides belong to two proteins, the DNA polymerase processivity factor (gene UL42, three peptides) and the tegument protein VP16 (gene UL48, two peptides). No significant motifs are shared between the HSV proteins and the human receptor, providing no support for molecular mimicry based on linear epitopes. Some H+N+ patients detected all five signature peptides, whereas others only subsets with a varying distribution and three patients displayed no reactivity (Figure 3H). A UL42/48 score that incorporated relative binding values from the five signature peptides identified H+N+ patients within the entire study cohort at a sensitivity of 75% and a specificity above 97%; with similar performance in CSF and in serum (Figures 3I-3K).

HSE patients with secondary development of NMDARE thus have specific features of the HSV antibody response. HSV1 antibodies displayed greater clonality, binding strength and increased degree of ITS, findings consistent with a prospective HSE cohort study published recently (Armangue et al., 2023). In that study, the investigators observed increased type I interferon responses 21 days after onset in 20% of the cases that went on to develop autoimmune encephalitis, mainly of the NMDARE type. This slower interferon activation decay indicates a prolonged antiviral immune response associated with increased antibody production. The NMDARE-associated HSV1 response reported here comprises antibodies recognizing peptides from the UL42 and UL48 HSV1 proteins. Both proteins inhibit NF-κB activation and thus abrogate the production of type I interferons. UL42 and UL48 antibodies, if able to reach their intracellular targets, might be expected to disinhibit this HSV1-protein mediated interferon suppression, thereby providing a potential mechanism for immune hyperactivation (and ensuing autoantibody development). Interestingly, HSV1 UL48 antibodies were previously described in 30% of Behçet's disease patients, an autoimmune systemic vasculitis, further supporting an immune activating role of these antibodies. This suggests treatments aiming to deplete or suppress antibody production may prevent or reduce disease symptoms in patients yet to exhibit clinical manifestations of autoimmune HSV-driven NMDARE identified by the method according to the invention.

The signature antibodies are promising prognostic biomarkers, since they were found in patients before and after NMDARE manifestation, equally in CSF and serum samples, up to >20 years after HSE. Collectively, these serum antibodies may provide a more sensitive, robust, and convenient biomarker, versus the relatively brief diagnostic window of the interferon signature (weeks).

*Methods*

*PhIP-Seq experiments*

For IgG antibody profiling, PhIP-Seq libraries displaying tiled proteomes, including those of human viruses (VirScan, 110,215 56-mer peptides). They were used following previously described PhIP-Seq protocols. In brief, for incubation of patients' IgG antibodies with PhIP-Seq libraries, CSF and serum samples were randomly assigned to four 96-well plates with equal distribution of cohorts across plates and by processing paired samples on the same plate. CSF and serum samples were diluted to 2 μg of IgG if possible, with rounded volumes capped at a maximum of 40 μL (49 CSF samples with 2 μg $\pm$ 20%, median 2.00 μg; 79 CSF samples with < 1.6 μg, median 0.81 μg, 25 CSF with unknown concentrations, 126 samples with 2 μg $\pm$ 20%, median 1.98 μg; 22 serum samples with unknown concentrations). The mixture was kept shaking overnight at 4°C, then incubated with protein A and protein G coated magnetic beads (Dyna beads, Invitrogen) to immunocapture phage bound to IgG. The captured phage library inserts were amplified for Illumina sequencing using two lanes of a NovaSeq SP 100 flow cell with estimated coverage of approximately 3.5 reads per peptide.

*PhIP-Seq analytics*

Fastq files were aligned to quantify peptide-corresponding read counts for all peptides, but excluding those representing HIV. For antibody reactivity evaluation, relative binding to each peptide was measured as fold-change versus bead-only ("mock IP") control conditions and P-values of differential abundance were calculated using the EdgeR software. Peptide reactivities were considered significant and referred to as a hit if the read count in the sample was at least 15, the relative binding was at least 5-fold over bead-controls, and the P-value was below 0.001. Samples were excluded from further analysis if total peptide hits were below 250 (two serum and one CSF sample) or maximum relative binding was below 40 (one additional serum). Correctness of CSF/serum sample pairing was evaluated by correlating public peptide reactivities PhIP-seq libraries(Angkeow et al., 2022)( and by excluding blatantly discordant pairs from further analysis if the Pearson's correlation coefficient was below 0.6. Reactivities to proteins were evaluated as maximum relative binding to a single peptide representing the respective protein. Reactivities to viruses were evaluated as mean of maximum relative binding to five peptides representing five proteins of the respective virus. To evaluate prevalence of antibody responses at the viral species level, virus aggregate reactivity scores (VARscores) were computed by comparing average relative binding of specific peptides to distributions of average randomly selected peptides and evaluated using the established threshold of positivity at 0.72.

*MICAR*

MICAR scoring for peptide reactivities uses paired relative binding values analogous to routine-diagnostic antibody indices.(Reiber & Lange, 1991) In this study MICAR scores were computed for relative binding value from pairs of CSF/serum (regular MICAR indices) or serum/CSF (inversed indices). MICAR scores are considered positive if i) the peptide reactivity is considered a hit in the pair's numerator (CSF for regular MICAR indices, serum for inversed indices), ii) the mean of the paired relative binding values is greater than three and ii) the ratio of the paired relative binding value is also greater than three. For normalization, the relative binding ratios for each peptide were adjusted by the ratio of median relative binding for the respective compartments. The median relative binding for both compartments of the ratio was calculated from relative binding values of all peptide hits from the numerator compartment (1145 ± 499 CSF hits for regular MICAR indices, 1637 ± 599 serum hits for inversed indices, mean ± standard deviation). When analyzing the relative binding values for both compartments separately (and not only its ratio), the normalization was applied by a normalization factor, defined as the square root of the ratio of median relative binding for the respective compartments (computed as above). Numerator binding values were divided by the normalization factor, denominator binding values were multiplied by the normalization factor. Of note, normalized relative binding can be below five for peptide hits. For MICAR scoring of aggregated virus reactivities, the mean of MICAR peptide scores was computed for all viruses with five or more peptide hits in the numerator compartment and was referred to as MICAR virus antibody index.

*Cited references:*

Altschul, S. F., et al. (1990). J Mol Biol, 215(3), 403-410.

Angkeow, J. et al. (2022). Immunity, 55(6), 1051-1066 e1054.

Armangue, T., et al. (2023). Brain, 146(10), 4306-4319.

Armangue, T., et al. (2018). Lancet Neurol, 17(9), 760-772.

Graus, F., et al. (2016). Lancet Neurol, 15(4), 391-404.

Hughes, et al. (2010). J Neurosci, 30(17), 5866-5875.

Kreye, J., et al. (2016). Brain, 139(Pt 10), 2641-2652.

Needleman, S. B., & Wunsch, C. D. (1970). J Mol Biol, 48(3), 443-453.

Nosadini, M., et al. (2021). JAMA Neurol, 78(11), 1333-1344.

Pearson, W. R., & Lipman, D. J. (1988) Proc Natl Acad Sci USA, 85(8), 2444-2448.

Planaguma, J., et al. (2015). Brain, 138(Pt 1), 94-109.

Pruss, H., et al. (2012). Ann Neurol, 72(6), 902-911.

Reiber, H., & Lange, P. (1991). Clin Chem, 37(7), 1153-1160.

Samanta, D., & Lui, F. (2024). Anti-NMDAR Encephalitis. In StatPearls.

Smith, T. F., & Waterman, M. S. (1981). J Mol Biol, 147(1), 195-197.

Titulaer, M. J., et al. Dalmau, J. (2013). Lancet Neurol, 12(2), 157-165.

All scientific publications and patent documents cited in the present specification are incorporated by reference herein.

*SEQUENCES:*

In the event of discrepancies between the sequences shown in the present specification and those of the enclosed sequence protocol according to WIPO standard ST.26, the sequences shown herein shall prevail.

SEQ ID NO 110 HSV UL42 393-488

TKRGRSGGEDARADTALKKPKTGSPTAPPPADPVPLDTEDDSDAADGTAARPAAPDARSGSRYACYFR
DLPTGEASPGAFSAFRGGPQTPYGFGFP

SEQ ID NO 2 HSV1 UL48 337-420

SEQ ID NO 002 HSV1 UL48 337-420

MVLIRAKLDSYSSFTTSPSEAVMREHAYSRARTKNNYGSTIEGLLDLPDDDAPEEAGLAAPRLSFLPA
GHTRRLSTAPPTDVSL

SEQ ID NO 003 HSV1 UL42 393-448 TKRGRSGGEDARADTALKKPKTGSPTAPPPADPVPLDTEDDSDAADGTA
ARPAAPD
SEQ ID NO 004 HSV1 UL42 421-476 PPADPVPLDTEDDSDAADGTAARPAAPDARSGSRYACYFRDLPTGEASP
GAFSAFR
SEQ ID NO 005 HSV1 UL42 449-488 ARSGSRYACYFRDLPTGEASPGAFSAFRGGPQTPYGFGFP
SEQ ID NO 006 HSV1 UL48 337-392 MVLIRAKLDSYSSFTTSPSEAVMREHAYSRARTKNNYGSTIEGLLDLPDD
DAPEEA
SEQ ID NO 007 HSV1 UL48 365-420 SRARTKNNYGSTIEGLLDLPDDDAPEEAGLAAPRLSFLPAGHTRRLSTAP
PTDVSL

**Claims**

1. A method to detect Herpes simplex virus Type 1 (HSV1)-specific antibodies, the method comprising measuring a level of antibodies specific for at least one HSV antigen in a biological sample,
   **characterized in that** the HSV antigen is selected from the list consisting of:

   ◦ a UL42 region polypeptide consisting of SEQ ID NO 001; or
   ◦ at least one UL42 peptide consisting of SEQ ID NO 003, SEQ ID NO 004, or SEQ ID NO 005; and/or
   ◦ a UL48 region polypeptide consisting of SEQ ID NO 002; or
   ◦ at least one UL48 peptide consisting of SEQ ID NO 006, or SEQ ID NO 007.

2. The method according to claim 1, wherein the method comprises measuring the level of antibodies specific for the UL42 region polypeptide as specified in claim 1.

3. The method according to claim 1 or 2, wherein the method comprises measuring the level of antibodies specific to at least one UL42 peptide as specified in claim 1.

4. The method according to any one of the preceding claims, wherein the method comprises measuring the level of antibodies specific for the UL42 peptides SEQ ID NO 3, SEQ ID NO 004, and SEQ ID NO 005.

5. The method according to any one of the preceding claims, wherein the method comprises measuring the level of antibodies specific for the UL48 region polypeptide as specified in claim 1.

6. The method according to any one of the preceding claims, wherein the method comprises measuring the level of antibodies specific for at least one UL48 peptide as specified in claim 1.

7. The method according to any one of the claims 1 to 4, or 6, wherein the method comprises measuring the level of antibodies specific for the UL48 peptides SEQ ID NO 006, and SEQ ID NO 007.

8. The method according to any one of the previous claims, wherein the level of antibodies bound to the at least one HSV antigen is determined by contacting the sample with a molecular probe specific for human immunoglobulin (Ig), said molecular probe being conjugated to a detectable label.

9. The method according to any one of the preceding claims, wherein method of measuring the level of antibodies comprises a detection methodology selected from the list consisting of passive agglutination, hemagglutination, western blot, enzyme-linked immunosorbent assay (ELISA), lateral flow device assay, fluorescence immunoassay, multiplexed flow cytometry immunoassay, luciferase immunoprecipitation assay, and radioimmunoassay

10. The method according to any one of the preceding claims, wherein the biological sample is a blood sample, particularly, wherein the biological sample is a plasma sample, or a serum sample.

11. The method according to any one of the claims 1 to 9, wherein the biological sample is a cerebral spinal fluid sample.

12. A method to predict the prognosis of a patient diagnosed with herpes simplex virus encephalitis comprising the steps of:

- performing the method as specified in any one of the claims 1 to 11 on a biological sample obtained from a patient;
- assigning the patient:

• a high likelihood of developing anti-N-methyl-D-aspartate receptor (NMDAR) encephalitis if the level of antibody specific for the at least one HSV antigen detected in the sample is equal to or above a threshold; or
• a low likelihood of developing anti-NMDAR encephalitis if the level of antibody specific for the at least one HSV antigen is below said threshold.

13. The method according to claim 12, wherein the sample is a serum sample, and a high likelihood of developing NMDAR encephalitis is defined as a more than 80% probability of developing NMDAR encephalitis in the next 2 years, and a low likelihood of developing NMDAR encephalitis is defined as a less than 5% probability of developing NMDAR encephalitis in the next 2 years.

14. The method according to claim 12 or 13, wherein the patient experienced HSV encephalitis symptom onset at least two weeks before the biological sample was obtained.

15. An immunomodulatory agent selected from the list consisting from an immunosuppressive agent or intravenous immunoglobulin, for use in a patient assigned a high likelihood of developing NMDAR encephalitis according to the method of any one of the claims 12 to 14, particularly wherein the immunomodulatory agent is an immunosuppressive agent selected from the list consisting of a corticosteroid, azathioprine, mycophenolate mofetil, azathioprine, bortezomib, cyclophosphamide, methotrexate, rituximab, alemtuzumab, and/or tocilizumab.

Fig. 1

Fig. 1 (continued)

**F**
Repertoire diversity (CSF)

**G**
Peptide reactivity (CSF)

**H**
Maximum reactivity (CSF)

**I**
Repertoire diversity (serum)

**J**
Peptide reactivity (serum)

**K**
Maximum reactivity (serum)

Fig. 2

A
Time point
ns

B
HSV1/HSV2 diversity

C
CSF Cell Count

D
HSV peptide reactivity (CSF)

E
HSV peptide ITS

Fig. 2 (continued)

Fig. 3

Europäisches
Patentamt

European
Patent Office

Office européen
des brevets

# EUROPEAN SEARCH REPORT

**Application Number**

EP 24 18 7247

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/132550 A1 (BRIGHAM & WOMENS HOSPITAL INC [US]; HARVARD COLLEGE [US]) 3 August 2017 (2017-08-03) * paragraph [0123] - paragraph [0132]; claim 1 * & DATABASE Geneseq [Online] 21 September 2017 (2017-09-21), "Human herpesvirus 1 peptide SEQ: 1896.", retrieved from EBI accession no. GSP:BEE64274 Database accession no. BEE64274 * sequence * & DATABASE Geneseq [Online] 21 September 2017 (2017-09-21), "Human herpesvirus 1 peptide SEQ: 1921.", retrieved from EBI accession no. GSP:BEE64299 Database accession no. BEE64299 * sequence * ----- | 1-11 | INV. G01N33/564 G01N33/569 G01N33/68 |
| X | WESTMAN GABRIEL ET AL: "Cerebrospinal fluid biomarkers of brain injury, inflammation and synaptic autoimmunity predict long-term neurocognitive outcome in herpes simplex encephalitis", CLINICAL MICROBIOLOGY AND INFECTION., vol. 27, no. 8, 1 August 2021 (2021-08-01), pages 1131-1136, XP093226283, United Kingdom, Switzerland ISSN: 1198-743X, DOI: 10.1016/j.cmi.2020.09.031 * page 1134 - page 1135 * * abstract * ----- -/-- | 12-15 | **TECHNICAL FIELDS SEARCHED (IPC)** G01N |

The present search report has been drawn up for all claims

| Place of search | Date of completion of the search | Examiner |
|---|---|---|
| The Hague | 21 November 2024 | Fabrowski, Piotr |

page 1 of 2

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MONACO DANIEL R. ET AL: "Deconvoluting virome-wide antibody epitope reactivity profiles", EBIOMEDICINE, vol. 75, 16 December 2021 (2021-12-16), page 103747, XP093225697, NL ISSN: 2352-3964, DOI: 10.1016/j.ebiom.2021.103747 * page 5 - page 11 * | 1-14 | |
| A | KAMEI S ET AL: "Evaluation of combination therapy using aciclovir and corticosteroid in adult patients with herpes simplex virus encephalitis", JOURNAL OF NEUROLOGY NEUROSURGERY & PSYCHIATRY., vol. 76, no. 11, 1 November 2005 (2005-11-01), pages 1544-1549, XP093226163, GB ISSN: 0022-3050, DOI: 10.1136/jnnp.2004.049676 * the whole document * | 15 | **TECHNICAL FIELDS SEARCHED (IPC)** |

The present search report has been drawn up for all claims

| Place of search | Date of completion of the search | Examiner |
|---|---|---|
| The Hague | 21 November 2024 | Fabrowski, Piotr |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 7247

This annex lists the patent family members relating to the patent documents cited in the above-mentioned European search report.
The members are as contained in the European Patent Office EDP file on
The European Patent Office is in no way liable for these particulars which are merely given for the purpose of information.

21-11-2024

| Patent document cited in search report | Publication date | Patent family member(s) | | Publication date |
|---|---|---|---|---|
| WO 2017132550 A1 | 03-08-2017 | EP | 3408284 A1 | 05-12-2018 |
| | | US | 2019055545 A1 | 21-02-2019 |
| | | US | 2022251541 A1 | 11-08-2022 |
| | | WO | 2017132550 A1 | 03-08-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SAMBROOK et al.** Molecular Cloning: A Laboratory Manual. Cold Spring Harbor Laboratory Press, 2012
- **AUSUBEL et al.** Short Protocols in Molecular Biology. John Wiley & Sons, Inc, 2002
- *CHEMICAL ABSTRACTS*, 9007-8304
- *CHEMICAL ABSTRACTS*, 53-03-2
- *CHEMICAL ABSTRACTS*, 83-43-2
- *CHEMICAL ABSTRACTS*, 44-86-6
- *CHEMICAL ABSTRACTS*, 128794-94-5
- *CHEMICAL ABSTRACTS*, 129324-69-7
- *CHEMICAL ABSTRACTS*, 50-18-0
- *CHEMICAL ABSTRACTS*, 59-05-2
- *CHEMICAL ABSTRACTS*, 174722-31-7
- *CHEMICAL ABSTRACTS*, 1299440-37-1
- *CHEMICAL ABSTRACTS*, 216503-57-0
- *CHEMICAL ABSTRACTS*, 357823-41-9
- **L. LACHMAN et al.** The Theory and Practice of Industrial Pharmacy,. 2013
- **ALTSCHUL, S. F et al.** *J Mol Biol*, 1990, vol. 215 (3), 403-410
- **ANGKEOW, J. et al.** *Immunity*, 2022, vol. 55 (6), 1051-1066 e1054
- **ARMANGUE, T. et al.** *Brain*, 2023, vol. 146 (10), 4306-4319
- **ARMANGUE, T. et al.** *Lancet Neurol*, 2018, vol. 17 (9), 760-772
- **GRAUS, F. et al.** *Lancet Neurol,*, 2016, vol. 15 (4), 391-404
- **HUGHES et al.** *J Neurosci,*, 2010, vol. 30 (17), 5866-5875
- **KREYE, J et al.** *Brain*, 2016, vol. 139, 2641-2652
- **NEEDLEMAN, S. B.** ; **WUNSCH, C. D.** *J Mol Biol*, 1970, vol. 48 (3), 443-453
- **NOSADINI, M. et al.** *JAMA Neurol*, 2021, vol. 78 (11), 1333-1344
- **PEARSON, W. R.** ; **LIPMAN, D. J.** *Proc Natl Acad Sci USA*, 1988, vol. 85 (8), 2444-2448
- **PLANAGUMA, J. et al.** *Brain*, 2015, vol. 138, 94-109
- **PRUSS, H. et al.** *Ann Neurol*, 2012, vol. 72 (6), 902-911
- **REIBER, H** ; **LANGE, P.** *Clin Chem*, 1991, vol. 37 (7), 1153-1160
- **SAMANTA, D.** ; **LUI, F.** Anti-NMDAR Encephalitis. *StatPearls*, 2024
- **SMITH, T. F** ; **WATERMAN, M. S**. *J Mol Biol*, 1981, vol. 147 (1), 195-197
- **TITULAER, M. J.** ; **DALMAU, J. et al.** *Lancet Neurol*, 2013, vol. 12 (2), 157-165